# EUROPÄISCHE PATENTANMELDUNG

(11) **EP 1 126 173 A1**
(43) Veröffentlichungstag der Anmeldung: **22.08.2001**
(21) Anmeldenummer: 01112768.5
(22) Anmeldetag: 03.07.1998
(51) Int. Cl.: F04B 53/10, F04B 39/10

(54) **Pumpe, insbesondere ein- oder mehrstufige Saugpumpe**

(30) Priorität: 30.07.1997 DE 19732808
(62) Teilanmeldung aus: 98943745.4
(71) Anmelder: KNF Neuberger GmbH, D-79112 Freiburg (DE)
(72) Erfinder: Becker, Erich, 79189 Bad Krozingen (DE); Riedlinger, Heinz, 28211 Bremen (DE)
(74) Vertreter: Maucher, Wolfgang, Dipl.-Ing.

(57) **Zusammenfassung**

Die Erfindung betrifft eine Pumpe, insbesondere eine ein- oder mehrstufige Saugpumpe, mit wenigstens einem, in einem Pumpenkopf befindlichen Einlaß- und zumindest einem Auslaßventil, von denen mindestens ein Ventil eine vom Fördermedium gesteuerte Ventilscheibe (12) aufweist, die in Schließstellung des Ventils (10, 11) an einer, zumindest eine Ventilöffnung (22) umgrenzenden Ventil-Dichtfläche (23) des Pumpenkopfes (7) dichtend anliegt. Bei einer derartigen Pumpe, die zumindest ein Ventil mit einer vom Fördermedium gesteuerten Ventilscheibe hat, können jedoch bereits geringe Flüssigkeitsmengen von beispielsweise ein oder zwei Tropfen dazu führen, daß die Ventilscheibe am Ventilsitz "festklebt" und daß die bei niedrigen absoluten Drücken entsprechend geringen Differenzdrücke nicht ausreichen, um die Ventilscheibe zu bewegen. Um einem derartigen "Klebeeffekt" entgegenzuwirken, ist erfindungsgemäß vorgesehen, daß die dichtenden Berührungsstellen zwischen der Dichtseite der Ventilscheibe (12) und der die Ventilöffnung (22) umgrenzenden Ventil-Dichtfläche (23) des Pumpenkopfes (7) im wesentlichen als linienförmige Berührungsstellen ausgebildet sind (vgl. Fig. 3).

## Beschreibung

Die Erfindung betrifft eine Pumpe, insbesondere eine ein- oder mehrstufige Saugpumpe, mit wenigstens einem, in einem Pumpenkopf befindlichen Einlaß- und zumindest einem Auslaßventil, von denen mindestens ein Ventil eine vom Fördermedium gesteuerte Ventilscheibe aufweist, die in Schließstellung des Ventils an einer, zumindest eine Ventilöffnung umgrenzenden Ventil-Dichtfläche des Pumpenkopfes dichtend anliegt.

Derartige Saugpumpen werden beispielsweise in Bearbeitungsvorrichtungen verwendet, die als Autoklaven zum Sterilisieren medizinischer Gebrauchsgegenstände ausgebildet sind. Diese vorbekannten Bearbeitungsvorrichtungen weisen eine luftdicht verschließbare Bearbeitungskammer auf, in welche die darin befindlichen Instrumente vor dem Sterilisationsvorgang zunächst einem sogenannten fraktionierten Vorvakuum ausgesetzt werden, bei dem durch wiederholtes Evakuieren der Luft im Wechsel mit dem periodischen Einströmen von Dampf eine besonders gute Luftentfernung auch aus englumigen Instrumenten erreicht wird. Während des Sterilisationsvorganges werden die Instrumente in der Bearbeitungs- oder Sterilisationskammer unter Überdruck heißem Wasserdampf ausgesetzt. Um die Instrumente nach dem Sterilisieren rasch und rückstandslos zu trocknen, wird in der Bearbeitungskammer anschließend wiederum ein sogenanntes Nachvakuum erzeugt, welches die Trocknungszeit des Sterilisiergutes verkürzen und den Trocknungsvorgang optimieren soll.

Zum Evakuieren ist die Sterilisationskammer solch vorbekannter Dampfsterilisationsvorrichtungen an eine Fördereinrichtung angeschlossen, die eine Vakuumpumpe aufweist. Wegen der Beaufschlagung der Vakuumpumpe mit Wasserdampf werden bislang nur Wasserringpumpen oder Membranpumpen verwendet. Wegen der Baugröße und der Nachteile einer Wasserringpumpe kommen in den beispielsweise für die Arztpraxis vorgesehenen kleineren Dampfsterilisationsvorrichtungen meist nur Membranpumpen in Frage.

Bei der Kopplung einer herkömmlichen Membran-Vakuumpumpe mit einem herkömmlichen Autoklaven tritt allerdings folgendes Problem auf: Nach Beendigung eines Sterilisiervorganges wird der Dampf über eine Verbindungsleitung durch die Vakuumpumpe ausgestoßen. In der kalten Verbindungsleitung und der kalten Vakuumpumpe kondensiert mindestens ein Teil des Dampfes. Dieses Kondensat wird von der Vakuumpumpe abgepumpt. Problematisch ist hierbei, daß während des Betriebs im Bereich des sich erwärmenden Pumpenkopfes eine Rückverdampfung des Kondensats auftreten kann, so daß dann ein entsprechend größeres Volumen abgepumpt werden muß. Dies erfordert eine erhebliche Zeit.

Doch selbst wenn es gelingt, eine Rückverdampfung des Kondensats im Bereich des sich erwärmenden Pumpenkopfes zu verhindern, so besteht doch das Problem, daß bereits geringe Flüssigkeitsmengen von beispielsweise ein oder zwei Tropfen dazu führen können, daß die Ventilscheibe am Ventilsitz "festklebt" und daß die bei niedrigen absoluten Drücken entsprechend geringen Differenzdrücke nicht ausreichen, um die Ventilscheibe zu bewegen.

Die erwähnten Probleme stellen sich über die eingangs beschriebenen Autoklaven hinaus auch bei allen anderen, beispielsweise zur Geltrocknung verwendeten Bearbeitungsvorrichtungen, aus deren Bearbeitungskammer ein feuchtes oder flüssiges Fördermedium zu evakuieren ist. Dabei soll hier unter einem feuchten Fördermedium beispielsweise jedes Gas oder Gasgemisch verstanden sein, welches zumindest eine Teilmenge eines in gas- oder dampfförmigem beziehungsweise tropfbar-flüssigem Zustand befindlichen Stoffes transportiert.

Es besteht daher insbesondere die Aufgabe, eine Pumpe der eingangs erwähnten Art, insbesondere eine ein- oder mehrstufige Saugpumpe, zu schaffen, bei der derartige "Klebeeffekte" auch beim Evakuieren feuchter Gase mit Sicherheit vermieden werden und die sich auch dann noch beispielsweise schwerkraftbedingt und/oder aufgrund ihrer Pumpwirkung selbständig entleeren kann.

Die erfindungsgemäße Lösung dieser Aufgabe besteht bei der Pumpe der eingangs erwähnten Art insbesondere darin, daß die dichtenden Berührungsstellen zwischen der Dichtseite der Ventilscheibe und der die Ventilöffnung umgrenzenden Ventil-Dichtfläche des Pumpenkopfes im wesentlichen als linienförmige Berührungsstellen ausgebildet sind. Durch diese linienförmigen Berührungsstellen wird eine flächige Auflage der Ventilscheibe vermieden, die andernfalls zu unerwünschten Klebeeffekten und - bei geringen Differenzdrücken - zu entsprechenden Störungen am Ventil führen könnten.

Um solche Klebeeffekte erforderlichenfalls auch auf der der Dichtseite der Ventilscheibe abgewandten Seite zu verhindern, ist es vorteilhaft, wenn die Berührungsstellen zwischen der der Dichtseite abgewandten Rückseite der Ventilscheibe einerseits und einer Auflage für die in Offenstellung befindliche Ventilscheibe andererseits im wesentlichen als punktförmige und/oder ebenfalls als linienförmige Berührungsstellen ausgebildet sind.

Zweckmäßig ist es, wenn die Dichtfläche im Pumpenkopf und/oder die Dichtseite der Ventilscheibe wenigstens einen, zumindest eine Ventilöffnung umgrenzenden Ringvorsprung mit vorzugsweise konisch sich zur Berührungsstelle hin verjüngendem Querschnitt aufweist. Dabei wird eine Ausführungsform bevorzugt, die an der Dichtfläche des Pumpenkopfes zumindest einen konischen Ringvorsprung hat.

Um auch auf der der Dichtseite abgewandten Rückseite der Ventilscheibe derartige Klebeeffekte zu vermeiden, ist es zweckmäßig, wenn die rückseitige Auflage für die in Offenstellung befindliche Ventilscheibe und/oder die Ventilscheibenrückseite eine Profilierung als linienförmige und/oder punktförmige Berührungsstellen zur Verkleinerung der Anlageoberfläche aufweist.

Dabei kann die rückseitige Auflage für die Ventilscheibe durch die Kanten eines treppenförmigen Vorsprungs oder mehrerer treppenförmiger Vorsprünge mit vorzugsweise linienförmigen Berührungsstellen gebildet sein.

Vorteilhaft ist es, wenn die Saugpumpe in ihren vom Fördermedium beaufschlagten Bereichen aus korrosionsbeständigen Werkstoffen hergestellt ist. Dadurch werden eine lange Lebensdauer der Pumpe sowie lange Service-Intervalle noch zusätzlich begünstigt.

Weitere Merkmale der Erfindung ergeben sich aus der folgenden Beschreibung der erfindungsgemäßen Ausführungsbeispiele in Verbindung mit den Ansprüchen sowie der Zeichnung. Die einzelnen Merkmale können je für sich oder zu mehreren bei einer Ausführungsform gemäß der Erfindung verwirklicht sein.

Es zeigt:
- Fig. 1: eine schematisch dargestellte und hier als Dampfsterilisationsvorrichtung ausgebildete Bearbeitungsvorrichtung mit einer Bearbeitungs- oder Sterilisationskammer, welche an eine Fördereinrichtung angeschlossen ist, die eine Vakuumpumpe zum Evakuieren der Bearbeitungs- oder Sterilisationskammer aufweist,
- Fig. 2: die Saugpumpe der Bearbeitungsvorrichtung gemäß Figur 1 in einem Teil-Querschnitt im Bereich des Einlaßventiles, wobei das Einlaßventil in seiner Offenstellung dargestellt ist, und
- Fig. 3: ein mit Figur 2 vergleichbares Einlaßventil für eine Bearbeitungsvorrichtung gemäß Figur 1.

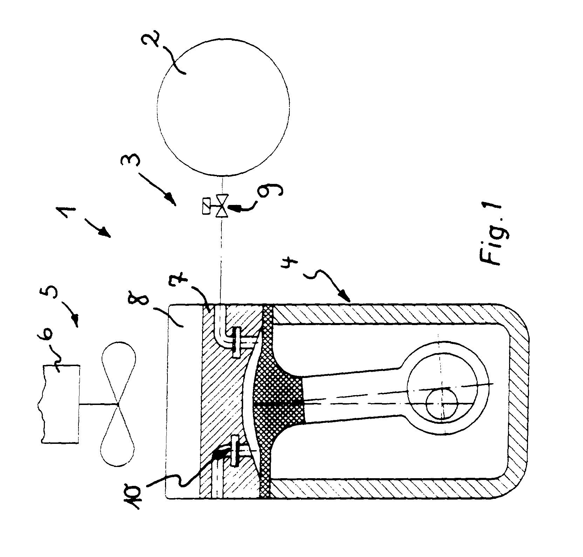

In Figur 1 ist eine als Dampfsterilisationsvorrichtung 1 ausgebildete Bearbeitungsvorrichtung dargestellt, die eine luftdicht verschließbare Bearbeitungs- oder Sterilisationskammer 2 aufweist. An die Sterilisationskammer 2 ist eine Fördereinrichtung 3 angeschlossen, die zum Evakuieren der Sterilisationskammer 2 dient. Diese Fördereinrichtung 3 weist eine hier als Membranpumpe ausgebildete Saugpumpe 4 auf, die sowohl Kondensat fördern wie auch Vakuum erzeugen kann.

Die Fördereinrichtung 3 ist zumindest in einem Teilbereich mittels einer Kühleinrichtung 5 derart kühlbar, daß sich das Fördermedium der Fördereinrichtung 3 in flüssigem Aggregatzustand befindet oder überführt wird.

Wie Figur 1 zeigt, weist die Kühleinrichtung 5 ein Kühlgebläse 6 auf. Der im Kühlstrom des Kühlgebläses 6 angeordnete Pumpenkopf 7 der Saugpumpe 4 weist außenseitig zur Kühlflächen-Vergrößerung sowie zur Strömungsführung des Kühlstromes Kühlrippen 8 auf.

Nachdem die zu sterilisierenden Instrumente und sonstigen medizinischen Gebrauchsartikel in die luftdicht verschließbare Sterilisationskammer 2 eingebracht wurden, wird die Sterilisationskammer 2 beispielsweise auf 200 Millibar evakuiert. Dabei kann erforderlichenfalls durch wiederholtes Evakuieren der in der Sterilisationskammer 2 befindlichen Luft im Wechsel mit dem periodischen Einströmen von Dampf eine besonders gute Luftentfernung auch aus englumigen Instrumenten und dergleichen erreicht werden.

Für den nachfolgenden Sterilisiervorgang wird das in die Strömungsführung zwischen Sterilisationskammer 2 und Vakuumpumpe 4 befindliche, vorzugsweise elektrisch betätigbare Schließventil 9 geschlossen, um die Sterilisationskammer 2 aufzuheizen und unter Überdruck mit Dampf zu füllen.

Nach Beendigung dieses Zyklus wird in der Sterilisationskammer 2 ein Nachvakuum erzeugt, damit das eventuell auf den Instrumenten und dergleichen kondensierte Wasser abdampfen und das Sterilisiergut völlig trocken werden kann. Dazu wird das Schließventil 9 zunächst geöffnet und der Dampf strömt, solange in der Sterilisationskammer 2 noch Überdruck herrscht, über die Saugpumpe 4 aus. Dabei kondensiert mindestens ein Teil des Dampfes in der Saugpumpe 4 und wird in flüssiger Form ins Freie befördert.

Wenn der Überdruck in der Sterilisationskammer 2 abgebaut ist, schließt das auch als Rückschlagventil dienende Auslaßventil 10 der Membranpumpe im Rhythmus des Pumpvorganges. Die Saugpumpe 4 zieht nun das dampfhaltige Fördermedium aus der Sterilisationskammer 2, wobei der mitgeführte Dampf beim Ausschieben in der Saugpumpe 4 auf Atmosphärendruck verdichtet wird und bei Kopftemperatur unter 100°C in der Pumpe kondensiert. Das Kondensat wird über die Saugpumpe 4 ins Freie befördert. In den Toträumen kann jedoch noch ein Rest des Kondensats verbleiben.

Beim Ansaughub wird der Druck im Arbeitsraum der Saugpumpe 4 reduziert. Zumindest ein Teil des in der Saugpumpe 4 verbliebenen Kondensats könnte nun verdampfen, wenn der Ansaugdruck in der Vakuumpumpe 4 den zur augenblicklichen Kopftemperatur am Pumpenkopf 7 entsprechenden Verdampfungsdruck unterschreiten würde. Eine solche Rückverdampfung des im Pumpenkopf 7 befindlichen Kondensats oder Kondensatfilms würde jedoch erneut zu einer Vergrößerung des abzupumpenden Fördervolumens und somit zur Reduzierung des Ansaugvolumens sowie der Saugleistung der Vakuumpumpe 4 führen.

Um eine solche Rückverdampfung auszuschließen, ist der Pumpenkopf 7 der Vakuumpumpe mittels der Kühleinrichtung 5 derart kühlbar, daß die Kopftemperatur etwa in oder vorzugsweise unter der Verdampfungs- oder Siedetemperatur liegt, die dem bei dessen Sterilisationsvorgang gewünschten Verdampfungsdruck in der Sterilisationskammer 2 entspricht.

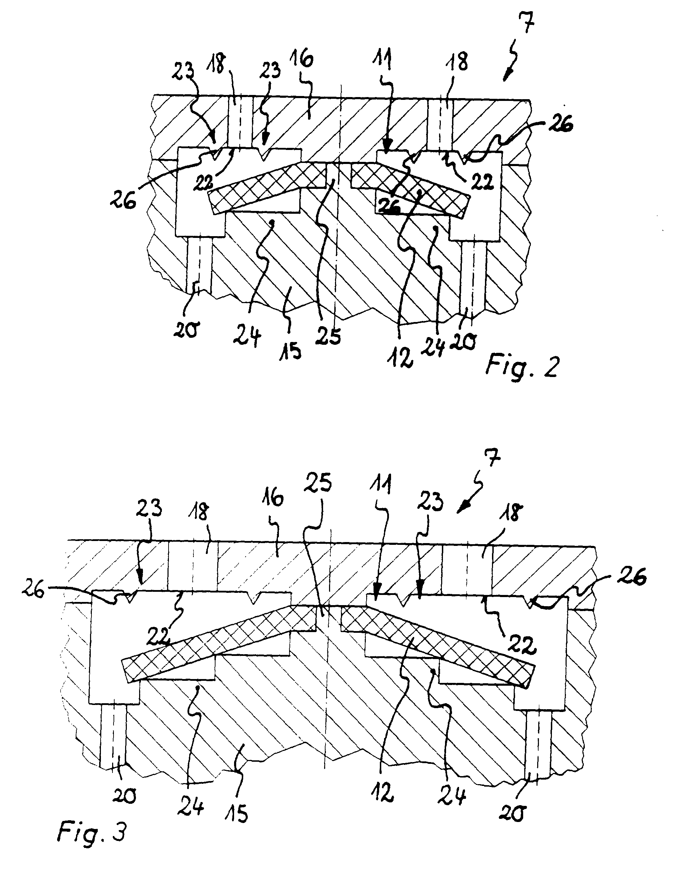

Wie bereits erwähnt, ist die Saugpumpe 4 der hier dargestellten Bearbeitsvorrichtung als Membranpumpe ausgebildet. Die Membranpumpe 4 hat Auslaß- und Einlaßventile 10, 11, welche jeweils eine vom Fördermedium gesteuerte Ventilscheibe 12 aufweisen. Das Einlaßventil 11 der Saugpumpe 4 ist in den Figuren 2 und 3 in zwei ähnlichen Ausführungen noch näher dargestellt.

Wie aus einem Vergleich der Figuren 1 sowie 2 und 3 deutlich wird, hat die Membranpumpe 4 ein Gehäuse 13, eine Membrane 14, einen Zwischendeckel 15 und einen sich daran anschließenden Abschlußdeckel 16. Die beiden Deckel 15, 16 bilden gemeinsam den Pumpenkopf 7. Im Abschlußdeckel 16 befindet sich ein Auslaßkanal 17 sowie ein über die Saugleitung 19 mit der Bearbeitungs- und Sterilisationskammer 2 verbundener Einlaßkanal 18. Die Ventile 10, 11 stehen über Zuleitungen 20 mit dem kalottenförmigen Pumpraum 21 im Zwischendeckel 15 in Verbindung.

In Schließstellung der Ventile 10, 11 liegt deren Ventilscheibe 12 dichtend an der die Ventilöffnung 22 umgrenzenden Ventildichtfläche 23 des Pumpenkopfes an. Um die Ventilöffnungsbewegung zu begrenzen, liegt die Ventilscheibe in der in Figur 2 und 3 gezeigten Offenstellung an einer als Anschlagfläche dienenden rückseitigen Auflage 24 an. Dabei sind die kreisscheibenförmigen Ventilscheiben 12 mittels eines Zapfens 25 zwischen dem Abschlußdeckel 16 und dem Zwischendeckel 15 zentral gehalten.

Die Eigenelastizität des Ventilscheiben-Werkstoffes sorgt üblicherweise für ein genügend schnelles Zurückgehen der Ventilscheibe 12 in ihre Schließstellung bei entsprechenden Druckdifferenzen des Fördermediums im Pumpbetrieb. Da mit Hilfe der auf den Pumpenkopf einwirkenden Kühleinrichtung 5 eine Rückverdampfung des im Pumpraum 21 oder den Ventilräumen befindlichen Kondensats oder Kondensatfilms vermieden wird, besteht bei Verwendung üblicher Saugpumpen die Gefahr, daß die Ventilscheiben 12 bereits bei geringen Flüssigkeitsmengen von einem oder zwei Tropfen an der Dichtfläche des Pumpenkopfes verklebt und daß die bei geringen absoluten Drücken vorhandenen kleinen Differenzdrücke nicht ausreichen, um die Adhäsionskräfte zu überwinden und die Ventilscheibe 12 zu bewegen.

Um ein einwandfreies Arbeiten der im Einlaß- und/oder im Auslaßventils 11, 10 vorgesehenen Ventilscheiben 12 zu gewährleisten, sind die dichtenden Berührungslinien zwischen der Dichtseite der Ventilscheibe 12 und der die zumindest eine Ventilöffnung 22 umgrenzenden Ventil-Dichtfläche 23 des Pumpenkopfes 7 im wesentlichen als linienförmige Berührungsstellen ausgebildet. Das in Figur 2 und 3 dargestellte Ventil 11 weist dazu zwei Ringvorsprünge 26 auf, die am Pumpenkopf 7 jeweils eine Ventilöffnung 22 umgrenzen. Dabei haben diese Ringvorsprünge 26 einen sich zur Berührungsstelle hin konisch verjüngenden Querschnitt.

Aus dem gleichen Grund sind die Berührungsstellen zwischen der Rückseite der Ventilscheibe 12 und der Auflage 24 für die in Offenstellung befindliche Ventilscheibe 12 hier im wesentlichen als linienförmige Berührungsstellen ausgebildet. Die rückseitige Auflage 24 der Ventile 10, 11 wird dazu durch die Kanten eines treppenförmigen Vorsprungs (vgl. Fig. 2) oder mehrerer treppenförmiger Vorsprünge (vgl. Fig. 3) mit vorzugsweise linienförmigen Berührungsstellen gebildet. Der Ventilscheibenrückseite können aber auch - hier nicht weiter dargestellte - punktförmige Vorsprünge als rückseitige Auflagen dienen.

Da die Ventilscheibe 12 der Ventile 10, 11 sowohl die Ventil-Dichtfläche 23 als auch die rückseitige Auflage 24 nur punkt- bzw. linienförmig berührt, können die infolge eines Kondensatfilms in den Ventilräumen auf die Ventilscheibe 12 einwirkenden Adhäsionskräfte so gering gehalten werden, daß die Ventilscheibe 12 auch bei geringen Differenzdrücken noch störungsfrei zwischen ihrer Offenstellung und der Schließstellung oszilliert. Zwar ist in den Figuren 2 und 3 nur jeweils das Einlaßventil 11 dargestellt, jedoch ist auch das Auslaßventil 10 der Bearbeitungs- oder Dampfsterilisationsvorrichtung 1 entsprechend ausgestaltet.

Die hier dargestellte Bearbeitungsvorrichtung erlaubt ein rasches und wirksames Abpumpen auch feuchter Fördermedien, wobei sich der Anwendungsbereich dieser Bearbeitungsvorrichtung nicht allein auf Dampfsterilisationsvorrichtungen beschränkt.

## Patentansprüche

1. Pumpe, insbesondere ein- oder mehrstufige Saugpumpe, mit wenigstens einem, in einem Pumpenkopf befindlichen Einlaß- und zumindest einem Auslaßventil, von denen mindestens ein Ventil eine vom Fördermedium gesteuerte Ventilscheibe (12) aufweist, die in Schließstellung des Ventils (10, 11) an einer, zumindest eine Ventilöffnung (22) umgrenzenden Ventil-Dichtfläche (23) des Pumpenkopfes (7) dichtend anliegt, **dadurch gekennzeichnet,** daß die dichtenden Berührungsstellen zwischen der Dichtseite der Ventilscheibe (12) und der die Ventilöffnung (22) umgrenzenden Ventil-Dichtfläche (23) des Pumpenkopfes (7) im wesentlichen als linienförmige Berührungsstellen ausgebildet sind.

2. Pumpe nach Anspruch 1, dadurch gekennzeichnet, daß die Berührungsstellen zwischen der Rückseite der Ventilscheibe (12) und einer Auflage (24) für die in Offenstellung befindliche Ventilscheibe (12) im wesentlichen als linienförmige und/oder punktförmige Berührungsstellen ausgebildet sind.

3. Pumpe nach Anspruch 1 oder 2, dadurch gekennzeichnet, daß die Ventil-Dichtfläche (23) und/oder die Dichtseite der Ventilscheibe (12) wenigstens einen, zumindest eine Ventilöffnung (22) umgrenzenden Ringvorsprung (26) mit vorzugsweise konisch sich zur Berührungsstelle verjüngendem Querschnitt aufweist.

4. Pumpe nach einem der Ansprüche 1 bis 3, dadurch gekennzeichnet, daß die rückseitige Auflage (24) für die in Offenstellung befindliche Ventilscheibe (12) und/oder Ventilscheibenrückseite als linienförmige und/oder punktförmige Berührungsstellen eine Profilierung zur Verkleinerung der Anlageoberfläche aufweist.

5. Pumpe nach einem der Ansprüche 1 bis 4, dadurch gekennzeichnet, daß die rückseitige Auflage (24) für die Ventilscheibe (12) durch die Kanten eines treppenförmigen Vorsprungs oder mehrerer treppenförmiger Vorsprünge mit vorzugsweise linienförmigen Berühungsstellen gebildet sind.
